# EUROPEAN PATENT APPLICATION

(11) **EP 2 305 307 A1**
(43) Date of publication of application: **06.04.2011**
(21) Application number: 09766596.2
(22) Date of filing: 12.06.2009
(51) Int. Cl.: A61K 45/00, A61K 9/06, A61K 9/08, A61K 9/10, A61K 9/12, A61K 9/14, A61K 9/70, A61K 31/165, A61K 31/192, A61K 31/245, A61K 31/381, A61K 31/405, A61K 31/5415, A61P 29/00

(54) **ANALGESIC ANTI-INFLAMMATORY PREPARATION FOR EXTERNAL APPLICATION**

(30) Priority: 16.06.2008 JP 2008156713
(71) Applicant: TEIKOKU SEIYAKU CO., LTD., Higashikagawa-shi, Kagawa 769-2695 (JP)
(72) Inventor: INOO, Katsuyuki, Higashikagawa-shi Kagawa 769-2695 (JP); KAWADA, Mitsuhiro, Higashikagawa-shi Kagawa 769-2695 (JP)
(74) Representative: Albrecht, Thomas
(86) International application number: PCT/JP2009/060768
(87) International publication number: WO 2009/154148

(57) **Abstract**

An external preparation that effectively produces the anti-inflammatory analgesic effect of a non-steroidal anti-inflammatory analgesic agent, and reduces skin irritation on the application site of the preparation and has an excellent effect on pain associated with inflammation such as chronic rheumatoid arthritis, osteoarthritis, and also lumbago is provided. The external preparation includes a non-steroidal anti-inflammatory analgesic agent and oxybuprocaine or a pharmaceutically acceptable salt thereof. The external preparation includes the non-steroidal anti-inflammatory analgesic agent at a content of 0.1 to 10 wt% based on the total weight of the formulation and oxybuprocaine or a pharmaceutically acceptable salt thereof at a content of 0.01 to 60 wt% based on the total weight of the formulation. The external preparation has a dosage form as an external preparation wherein the dosage form is an ointment, a solution, a suspension, an emulsion, a lotion, a cataplasm, a tape, an aerosol, or a powder for external use.

## Description

### TECHNICAL FIELD

The present invention relates to an anti-inflammatory analgesic preparation, and in particular, to an external preparation that includes a non-steroidal anti-inflammatory analgesic agent and a local anesthetic, oxybuprocaine, or a pharmaceutically acceptable salt thereof as medicinal ingredients and has a significantly improved anti-inflammatory analgesic effect.

### BACKGROUND ART

Many non-steroidal anti-inflammatory analgesic agents have been previously developed to replace steroidal anti-inflammatory analgesic agents, which have many side effects, and have come to be used clinically.
Although these non-steroidal anti-inflammatory analgesic agents produce an excellent anti-inflammatory analgesic effect, oral administration of these agents causes a serious side effect, a gastrointestinal disorder. Accordingly, transdermal absorption preparations to achieve an administration route with reduced side effects have been investigated. For example, an ointment, a plaster (a stick formulation), a cataplasm, a tape, solutions such as a suspension, an emulsion, and a lotion, an aerosol, and the like have been developed as an external preparation that includes a non-steroidal anti-inflammatory analgesic agent. Some formulations of these have already been used clinically.

However, non-steroidal anti-inflammatory analgesic agents are generally not absorbed transdermally very well, and the effect tends to be reduced when administered as an external preparation, as compared to the effect when administered orally.
Thus, various attempts to enhance the transdermal absorption have been made, such as increase of the content of the non-steroidal anti-inflammatory analgesic agent as an active ingredient in the formulation and addition of a transdermal absorption enhancer.

Recently, addition of a local anesthetic to a non-steroidal anti-inflammatory analgesic agent to enhance transdermal absorption and the like has been proposed as one of the attempts (Patent Documents 1 to 6).
For example, Patent Document 1 proposes a patch for external use that includes a local anesthetic such as lidocaine or benzocaine together with a non-steroidal anti-inflammatory analgesic agent such as indomethacin and ketoprofen. This patch is considered as a patch for external use that has an excellent analgesic effect particularly on pain associated with inflammation such as chronic rheumatoid arthritis, osteoarthritis, and lumbago.

Furthermore, Patent Document 2 proposes an external preparation that includes diclofenac sodium and a local anesthetic such as lidocaine or benzocaine, and Patent Document 3 proposes an external preparation that includes lidocaine together with piroxicam. Furthermore, Patent Documents 4 to 6 propose a patch for external use that includes a local anesthetic such as lidocaine or tetracaine together with a non-steroidal anti-inflammatory analgesic agent such as indomethacin and diclofenac.

The external preparations proposed above are considered as external preparations that are intended to, for example, reduce skin irritation caused by a non-steroidal anti-inflammatory analgesic agent or enhance permeation and diffusion of the drug in tissues, and effectively produce the anti-inflammatory analgesic effect of the non-steroidal anti-inflammatory analgesic agent.
Patent Document 1: International Publication WO 01/47559
Patent Document 2: Japanese Patent Application Laid-Open No. 2003-335663
Patent Document 3: Japanese Patent Application Laid-Open No. 2004-123632
Patent Document 4: Japanese Patent Application Laid-Open No. 2004-323502
Patent Document 5: Japanese Patent Application Laid-Open No. 2005-068035
Patent Document 6: Japanese Patent Application Laid-Open No. 2005-145932

However, in the case of actual dermal application, the non-steroidal anti-inflammatory analgesic agents in these external preparations do not have a very high level of anti-inflammatory analgesic effect, and further improvement is currently required.

### DISCLOSURE OF THE INVENTION

### PROBLEMS TO BE SOLVED BY THE INVENTION

In view of the above circumstances, the present inventors' object is to provide an external preparation that effectively produces the anti-inflammatory analgesic effect of a non-steroidal anti-inflammatory analgesic agent, and reduces skin irritation on the application site of the preparation and has an excellent effect on pain associated with inflammation such as chronic rheumatoid arthritis, osteoarthritis, and also lumbago.

### MEANS FOR SOLVING THE PROBLEMS

The present inventors conducted a diligent examination to solve the above-mentioned problems. The present inventors investigated previously proposed external preparations that included a non-steroidal anti-inflammatory analgesic agent and a local anesthetic and examined the effect obtained by including, in particular, the local anesthetic. Consequently, the present inventors found that the analgesic anti-inflammatory effect of a non-steroidal anti-inflammatory analgesic agent was enhanced effectively when oxybuprocaine, among other local anesthetics, was used in combination with the non-steroidal anti-inflammatory analgesic agent.

Specifically, the present inventors prepared an analgesic/antipruritic external preparation that included a local anesthetic, oxybuprocaine, together with a non-steroidal anti-inflammatory analgesic agent, and applied this formulation to an affected skin area with pain or an affected skin area with inflammation and pain. Consequently, the present inventors found that skin irritation was reduced and additionally, a very high level of analgesic anti-inflammatory effect was produced in addition to analgesic action exerted by the local anesthetic, oxybuprocaine. Thus, the present inventors accomplished the present invention.

Accordingly, a basic embodiment of the present invention is an external preparation that includes a non-steroidal anti-inflammatory analgesic agent and oxybuprocaine or a pharmaceutically acceptable salt thereof as medicinal ingredients.

Specifically, the present invention is an external preparation that includes the non-steroidal anti-inflammatory analgesic agent at a content of 0.1 to 10 wt% based on the total weight of the drug-containing preparation. The present invention is also an external preparation that includes oxybuprocaine or a pharmaceutically acceptable salt thereof at a content of 0.01 to 60 wt% based on the total weight of the drug-containing preparation.

More specifically, the present invention is an external preparation that includes 0.1 to 10 parts by weight of oxybuprocaine or a pharmaceutically acceptable salt thereof relative to 1 part by weight of the non-steroidal anti-inflammatory analgesic agent.

The present invention is also, specifically, an external preparation in which the non-steroidal anti-inflammatory analgesic agent to be included is one selected from indomethacin, ketoprofen, piroxicam, felbinac, bufexamac, suprofen, flurbiprofen, diclofenac, ibuprofen, and pharmacologically acceptable salts thereof.

Furthermore, the present invention is the above-mentioned external preparation whose dosage form as an external preparation is an ointment, a solution, a suspension, an emulsion, a lotion, a cataplasm, a tape, an aerosol, or a powder for external use.

Among them, one of the most preferable embodiments of the present invention is an external preparation in the form of a cataplasm or a tape that includes oxybuprocaine or a pharmaceutically acceptable salt thereof together with felbinac serving as a non-steroidal anti-inflammatory analgesic agent, as active ingredients.

### EFFECTS OF THE INVENTION

The present invention provides an external preparation that effectively produces the anti-inflammatory analgesic effect of a non-steroidal anti-inflammatory analgesic agent, and reduces skin irritation on the application site of the preparation and has an excellent effect on pain associated with inflammation.
The external preparation provided by the present invention also has excellent transdermal absorption and additionally has excellent permeation and diffusion of the active ingredients into tissues after its transfer into a living body. Therefore, the present invention provides external preparations in various dosage forms, which have a sufficient therapeutic effect on various types of pain and itching of the skin, in addition to action exerted by oxybuprocaine and have very few side effects. These external preparations have great medical value.

Specifically, the anti-inflammatory analgesic external preparation of the present invention is very effective against, for example, not only chronic pain such as chronic rheumatoid arthritis, osteoarthritis, and lumbago; inflammatory diseases such as periarthritis scapulohumeralis and tendovaginitis; diseases associated with pain such as pain resulting from a surgery, a trauma, and the like; but also atopic dermatitis, eczema, contact dermatitis, seborrheic dermatitis, urticaria, strophulus infantum, insect sting, cutaneous pruritus; itching associated with metabolic diseases such as uremia and chronic renal failure, endocrine diseases such as diabetes, and the like; and diseases associated with itching, for example itching associated with skin wounds such as cut wounds, postoperative wounds, and burn wounds; or neuropathic pain.

### BEST MODE FOR CARRYING OUT THE INVENTION

As described above, the basic embodiment of the present invention is an external preparation that includes a non-steroidal anti-inflammatory analgesic agent and oxybuprocaine as medicinal ingredients.
Non-steroidal anti-inflammatory analgesic agents that are included as an active ingredient may include, but are not limited to, compounds selected from indomethacin, ketoprofen, piroxicam, felbinac, bufexamac, suprofen, flurbiprofen, diclofenac, ibuprofen, and pharmacologically acceptable salts thereof.
These non-steroidal anti-inflammatory analgesic agents may also be used alone or used in a combination of two or more thereof.
In particular, it was found that selecting felbinac as a non-steroidal anti-inflammatory analgesic agent provided a very effective external preparation.

Although the content of the above-mentioned non-steroidal anti-inflammatory analgesic agent varies depending on the non-steroidal anti-inflammatory analgesic agent to be used and the desired dosage form of the external preparation, it is preferably 0.1 to 10 wt%, and more preferably 0.2 to 5 wt% based on the total weight of the drug-containing formulation.
Including a non-steroidal anti-inflammatory analgesic agent in an amount less than the above range is not preferable since the effect is insufficient. Including a non-steroidal anti-inflammatory analgesic agent in an amount more than the above range is also not preferable since the effect is not further enhanced and conversely side effects may be caused.

The oxybuprocaine that is to be included together with these non-steroidal anti-inflammatory analgesic agents is a drug that was developed as a local anesthetic, has surface anesthesia action, infiltration anesthesia action, and conduction anesthesia action, and is mainly used for surface anesthesia in the ophthalmologic field, for example.
In the present invention, it was found that the analgesic effect of a non-steroidal anti-inflammatory analgesic agent at the application site was synergistically enhanced due to the local anesthetic effect of oxybuprocaine, when the oxybuprocaine was included together with the non-steroidal anti-inflammatory analgesic agent. It was also found that skin irritation was reduced and additionally transdermal absorption of the non-steroidal anti-inflammatory analgesic agent in the external preparation was enhanced.

The content of oxybuprocaine or pharmaceutically acceptable salts thereof varies depending on the type of the non-steroidal anti-inflammatory analgesic agent which is included together and is not necessarily limited; however, the content is preferably 0.01 to 60 wt%, and more preferably 0.1 to 30 wt% based on the total weight of the drug-containing formulation.
Including oxybuprocaine or pharmaceutically acceptable salts thereof in an amount less than the above range is not preferable since the combined effect is insufficient. Including oxybuprocaine or pharmaceutically acceptable salts thereof in an amount more than the above range is also not preferable since the physical properties of the formulation are affected and side effects may be caused.

Furthermore, in the present invention, an external preparation that, while keeping the contents within the above-mentioned range, further included 0.1 to 10 parts by weight of oxybuprocaine or pharmaceutically acceptable salts thereof relative to 1 part by weight of the non-steroidal anti-inflammatory analgesic agent was found to be more effective.

The external preparation provided by the present invention is not particularly limited as long as its dosage form allows direct administration of an active ingredient to the surface of an affected skin area. For example, formulations such as an ointment, a solution (a suspension, an emulsion, a lotion, etc.), a cataplasm, a tape, an aerosol, and a powder for external use may be prepared and used.
In preparing these formulations, various ingredients that are used to prepare ordinary external preparations may be selected and used as appropriate, in addition to the non-steroidal anti-inflammatory analgesic agent and oxybuprocaine included as active ingredients.

Such ingredients, in the case of an ointment, a cream, a gel, and a lotion, may include bases such as white petrolatum, yellow petrolatum, lanolin, white beeswax, cetanol, stearyl alcohol, stearic acid, hydrogenated oil, hydrocarbon gel, polyethylene glycol, liquid paraffin, and squalane; solvents and solubilizers such as oleic acid, isopropyl myristate, glyceryl triisooctanoate, crotamiton, diethyl sebacate, diisopropyl adipate, hexyl laurate, fatty acids, fatty acid esters, aliphatic alcohols, and vegetable oil; antioxidants such as tocopherol derivatives, L-ascorbic acid, dibutylhydroxytoluene, and butylated hydroxyanisole; antiseptics such as p-hydroxybenzoic acid esters; humectants such as glycerin, propylene glycol, and sodium hyaluronate; surfactants such as polyoxyethylene derivatives, glycerine fatty acid esters, sucrose fatty acid esters, sorbitan fatty acid esters, propylene glycol fatty acid esters, and lecithin; thickeners such as carboxyvinyl polymers, xanthan gum, carboxymethylcellulose, carboxymethylcellulose sodium salts, hydroxypropylcellulose, and hydroxypropylmethylcellulose; and the like.
Additionally, a stabilizer, a preservative, an absorbefacient, a pH adjustor, and other suitable additives may be blended if desired.

In the case of a cataplasm, such ingredients may include tackifiers such as polyacrylic acid and polyacrylate copolymers; cross-linking agents such as aluminum sulfate, aluminum potassium sulfate, aluminum chloride, magnesium aluminometasilicate, and dihydroxyaluminum acetate; thickeners such as sodium polyacrylate, polyvinyl alcohol, polyvinylpyrrolidone, gelatin, sodium alginate, carboxymethylcellulose, carboxymethylcellulose sodium salts, hydroxypropylcellulose, and hydroxypropylmethylcellulose; polyalcohols such as glycerin, polyethylene glycol (macrogol), propylene glycol, and 1,3-butanediol; surfactants such as polyoxyethylene derivatives; perfumes such as 1-menthol; antiseptics such as p-hydroxybenzoic acid esters; purified water; and the like.
Additionally, a stabilizer, a preservative, an absorbefacient, a pH adjustor, and other suitable additives may be blended if desired.

In the case of a tape, an adhesive such as styrene-isoprene-styrene block copolymers (SIS block copolymers) and acrylic resin; a tackifier resin such as alicyclic saturated hydrocarbon resin, rosin-based resin, and terpene-based resin; a softener such as liquid rubber and liquid paraffin; an antioxidant such as dibutylhydroxytoluene; a polyalcohol such as propylene glycol; an absorbefacient such as oleic acid; a surfactant such as polyoxyethylene derivatives; and other suitable additives may be blended.
Furthermore, a water-containing tape may be formulated by adding polymers such as sodium polyacrylate and polyvinyl alcohol, which can retain water, and a small amount of purified water.
In this case, additionally, a stabilizer, a preservative, an absorbefacient, a pH adjustor, and other suitable additives may also be blended if desired.

In the case of an aerosol, a base such as white petrolatum, yellow petrolatum, lanolin, white beeswax, cetanol, stearyl alcohol, stearic acid, hydrogenated oil, hydrocarbon gel, polyethylene glycol, liquid paraffin, and squalane; a solvent and a solubilizer such as oleic acid, isopropyl myristate, diisopropyl adipate, isopropyl sebacate, glyceryl triisooctanoate, crotamiton, diethyl sebacate, hexyl laurate, fatty acids, fatty acid esters, aliphatic alcohols, and vegetable oil; an antioxidant such as tocopherol derivatives, L-ascorbic acid, dibutylhydroxytoluene, and butylated hydroxyanisole; antiseptics such as p-hydroxybenzoic acid esters; humectants such as glycerin, propylene glycol, and sodium hyaluronate; a surfactant such as polyoxyethylene derivatives, glycerine fatty acid esters, sucrose fatty acid esters, sorbitan fatty acid esters, propylene glycol fatty acid esters, and lecithin; a thickener such as carboxyvinyl polymers, xanthan gum, carboxymethylcellulose, carboxymethylcellulose sodium salts, hydroxypropylcellulose, and hydroxypropylmethylcellulose, which are used for preparation of an ointment, a cream, a gel, a suspension, an emulsion, a solution, a lotion, and the like; and moreover, various stabilizers, buffering agents, flavoring agents, suspending agents, emulsifiers, perfuming agents, preservatives, solubilizers, and other suitable additives may be blended.

In the case of a powder for external use, excipients such as potato starch, rice starch, cornstarch, talc, and zinc oxide or other suitable additives may be blended.
In this case, additionally, various stabilizers, preservatives, absorbefacients, and other suitable additives may also be blended if desired.

The procedure of preparing the external preparation provided by the present invention is not particularly limited. The external preparation of the present invention is produced using a method of producing an ordinary external preparation such as by thoroughly kneading the ingredients and base ingredients as needed, wherein the method depends on the desired dosage form.
In the preparation of a cataplasm and a tape, they may be prepared by spreading a kneaded mixture on a release liner, drying it, furthermore laminating it to a flexible backing layer, and cutting it to a desired size.

For example, when the external preparation provided by the present invention is an ointment, a solution (a suspension, an emulsion, a lotion, and the like), an aerosol, or a powder for external use, it is used by an ordinary method of use; for example, it is directly applied, for example by application to an affected skin area, or it is applied by using a backing layer such as a cloth coated or impregnated with the preparation.
A cataplasm or a tape is used by a method of directly applying these formulations to an affected skin area.

### [Examples]

Hereinbelow, the external preparation provided by the present invention will be described with reference to examples and test examples, but the present invention is not intended to be limited to these examples in any way.

### Example 1:

A drug-containing base having the formulation shown in Table 1 below was prepared. Specifically, felbinac was dissolved in crotamiton and oxybuprocaine was dissolved in propylene glycol. Both solutions were mixed, then these solutions were kneaded with the other ingredients shown in Table 1 until the mixture became homogeneous, thereby obtaining a drug-containing base. The drug-containing base thus prepared was spread onto a nonwoven fabric at 1000 g/m² and a polypropylene liner was applied thereto, and the resultant material was cut to a size of 10 x 14 cm² to obtain a patch for external use.

**[Table 1]**

| Ingredients | Contents |
|---|---|
| Felbinac | 0.5 |
| Oxybuprocaine | 7 |
| Propylene glycol | 5 |
| Glycerin | 10 |
| 70% sorbitol solution | 15 |
| Sodium polyacrylate | 5 |
| Carboxymethylcellulose sodium | 5 |
| Dihydroxyaluminum acetate | 0.2 |
| Diethanolamine | 0.2 |
| Crotamiton | 2 |
| Tartaric acid | 1 |
| Purified water | Balance |
| Total | 100 |

| | |
|---|---|
| Unit: part by weight | |

### Example 2:

A drug-containing base having the formulation shown in Table 2 below was prepared. Specifically, indomethacin was dissolved in crotamiton and oxybuprocaine was dissolved in propylene glycol.
Then, both of these solutions were kneaded with the other ingredients shown in Table 2 until the mixture became homogeneous, thereby obtaining a drug-containing base. The drug-containing base thus prepared was spread onto a nonwoven fabric at 1000 g/m² and a polypropylene liner was applied thereto, and the resultant material was cut to a size of 10 x 14 cm² to obtain a patch for external use.

**[Table 2]**

| Ingredients | Contents |
|---|---|
| Indomethacin | 0.5 |
| Oxybuprocaine | 5 |
| Propylene glycol | 10 |
| Crotamiton | 2 |
| Castor oil | 0.5 |
| Glycerin | 15 |
| Polyacrylic acid | 4 |
| Partially neutralized polyacrylic acid | 5 |
| Carboxymethylcellulose sodium | 4 |
| Aluminum hydroxide | 0.5 |
| Magnesium aluminometasilicate | 0.03 |
| Tartaric acid | 0.5 |
| Edetate disodium | 0.04 |
| Purified water | Balance |
| Total | 100 |

| | |
|---|---|
| Unit: part by weight | |

### Example 3:

A drug-containing base having the formulation shown in Table 3 below was prepared. Specifically, diclofenac sodium was dissolved in N-methyl-2-pyrrolidone and oxybuprocaine was dissolved in propylene glycol.
Then, both of these solutions were kneaded with the other ingredients shown in Table 3 until the mixture became homogeneous, thereby obtaining a drug-containing base. The drug-containing base thus prepared was spread onto a nonwoven fabric at 1000 g/m² and a polypropylene liner was applied thereto, and the resultant material was cut to a size of 10 x 14 cm² to obtain a patch for external use.

**[Table 3]**

| Ingredients | Contents |
|---|---|
| Diclofenac sodium | 1 |
| Oxybuprocaine | 5 |
| Propylene glycol | 10 |
| N-methyl-2-pyrrolidone | 5 |
| 70% sorbitol solution | 20 |
| Sodium polyacrylate | 5 |
| Carboxymethylcellulose sodium | 4 |
| Dried aluminum hydroxide gel | 0.3 |
| Tartaric acid | 2.5 |
| Kaoline | 5 |
| Purified water | Balance |
| Total | 100 |

| | |
|---|---|
| Unit: part by weight | |

### Example 4:

Based on the formulation shown in Table 4 below, a styreneisoprene-styrene block copolymer (SIS block copolymer), a hydrogenated rosin glycerol ester, liquid paraffin, polybutene, an antioxidant, and the like were added, and mixed and dissolved using toluene. Felbinac and oxybuprocaine were added to the mixture and mixed, and the mixture obtained by thorough kneading was spread on a release liner, and then toluene was evaporated. The mixture spread on the release liner was laminated to a flexible backing layer and cut to a desired size to obtain a tape.

**[Table 4]**

| Ingredients | Contents |
|---|---|
| Felbinac | 5 |
| Oxybuprocaine | 10 |
| Crotamiton | 5 |
| SIS block copolymer | 30 |
| Hydrogenated rosin glycerol ester | 30 |
| Polybutene | 5 |
| Liquid paraffin | 14 |
| Dibutylhydroxytoluene | 1 |
| Total | 100 |

| | |
|---|---|
| Unit: part by weight | |

### Comparative Example 1:

A patch for external use was produced by the same method as described in Example 1 except that an amount of purified water equal to that of oxybuprocaine was included instead of oxybuprocaine.

### Comparative Example 2:

A patch for external use was produced by the same method as described in Example 1 except that an amount of purified water equal to that of felbinac was included instead of felbinac.

### Comparative Example 3:

A patch for external use was produced by the same method as described in Example 1 except that an amount of purified water equal to that of felbinac and oxybuprocaine was included instead of them.

### Test Example:

The patches for external use obtained in Example 1 and Comparative Examples 1 to 3 were applied to the affected areas of 10 male volunteers, all of whom had low back pain, and then a sensory test was carried out.
The patches were applied for 12 hours a day over 7 days.
After the test was completed, the volunteers were asked to rank the efficacy as four grades, "complete response", "effective", "uncharged", and "deterioration."
Furthermore, similar tests were repeated after one week of the washout period until the evaluation of all the patches was completed.
The results are shown in Table 5 below.

**[Table 5]**

| | Numbers of volunteer | | | |
|---|---|---|---|---|
| | Example | Comparative Example | | |
| | 1 | 1 | 2 | 3 |
| Complete response | 8 | 4 | 0 | 0 |
| Effective | 1 | 5 | 3 | 1 |
| Unchanged | 1 | 1 | 7 | 8 |
| Deterioration | 0 | 0 | 0 | 1 |

As is shown in the table above, when the patch from Example 1, which was the inventive patch for external use that included both a non-steroidal anti-inflammatory analgesic agent, felbinac, and oxybuprocaine as active ingredients, was used, the percentage of improved cases (effective or better) was 90% (9/10). This percentage was similar to the percentage of improved cases when the patch from Comparative Example 1 that included no oxybuprocaine was used, which was 90% (9/10). However, the percentage of improved cases ranked as complete response was significantly different, which were 80% (8/10) and 40 % (4/10), respectively. Thus, the effectiveness of the present invention is understood.

### INDUSTRIAL APPLICABILITY

As described above, the present invention provides external preparations in various dosage forms, which effectively produce the anti-inflammatory analgesic effect of a non-steroidal analgesic anti-inflammatory agent, and reduce skin irritation on the application site of the preparation and have an excellent effect on pain associated with inflammation such as chronic rheumatoid arthritis, osteoarthritis, and also lumbago.
The external preparation provided by the present invention has excellent transdermal absorption and additionally has excellent permeation and diffusion of the active ingredients into tissues after its transfer into a living body, and has very few side effects. Therefore, the external preparation has great medical value.

## Claims

1. An external preparation, comprising a non-steroidal anti-inflammatory analgesic agent, and oxybuprocaine or a pharmaceutically acceptable salt thereof as medicinal ingredients.

2. The external preparation according to claim 1, wherein a content of the non-steroidal anti-inflammatory analgesic agent is 0.1 to 10 wt% based on a total weight of a drug-containing preparation.

3. The external preparation according to claim 1, wherein a content of oxybuprocaine or the pharmaceutically acceptable salt thereof is 0.01 to 60 wt% based on a total weight of a drug-containing preparation.

4. The external preparation according to claim 1, wherein 0.1 to 10 parts by weight of oxybuprocaine or the pharmaceutically acceptable salt thereof is contained relative to 1 part by weight of the non-steroidal anti-inflammatory analgesic agent.

5. The external preparation according to any of claims 1 to 4, wherein the non-steroidal anti-inflammatory analgesic agent is one selected from indomethacin, ketoprofen, piroxicam, felbinac, bufexamac, suprofen, flurbiprofen, diclofenac, ibuprofen, and pharmacologically acceptable salts thereof.

6. The external preparation according to any of claims 1 to 4, wherein the non-steroidal anti-inflammatory analgesic agent is felbinac.

7. The external preparation according to any of claims 1 to 6, being in a dosage form of an ointment, a solution, a suspension, an emulsion, a lotion, a cataplasm, a tape, an aerosol, or a powder for external use.

8. The external preparation according to claim 7, comprising felbinac and oxybuprocaine or the pharmaceutically acceptable salt thereof as medicinal ingredients.
